(19) Europäisches Patentamt
European Patent Office
Office européen des brevets

(11) **EP 3 535 573 B1**

(12) **EUROPÄISCHE PATENTSCHRIFT**

(45) Veröffentlichungstag und Bekanntmachung des Hinweises auf die Patenterteilung:
**17.09.2025 Patentblatt 2025/38**

(21) Anmeldenummer: **17777209.2**

(22) Anmeldetag: **21.09.2017**

(51) Internationale Patentklassifikation (IPC):
*G01N 29/04* (2006.01)  *G01N 23/18* (2018.01)
*G01N 29/11* (2006.01)  *G01N 29/265* (2006.01)
*G01N 29/27* (2006.01)  *G01N 29/275* (2006.01)
*G01N 29/44* (2006.01)  *G01N 21/88* (2006.01)
*G01N 22/02* (2006.01)

(52) Gemeinsame Patentklassifikation (CPC):
**G01N 29/265; G01N 21/95; G01N 29/043; G01N 29/11; G01N 29/27; G01N 29/275; G01N 29/4454; G01N 33/2022;** G01N 2291/0289

(86) Internationale Anmeldenummer:
**PCT/EP2017/073862**

(87) Internationale Veröffentlichungsnummer:
**WO 2018/082841 (11.05.2018 Gazette 2018/19)**

(54) **VERFAHREN ZUR DETEKTION VON MATERIALINHOMOGENITÄTEN**

METHOD FOR DETECTING MATERIAL INHOMOGENEITIES

PROCÉDÉ DE DÉTECTION D'INHOMOGÉNÉITÉS DE MATÉRIAU

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.11.2016 DE 102016221730**

(43) Veröffentlichungstag der Anmeldung:
**11.09.2019 Patentblatt 2019/37**

(73) Patentinhaber: **Robert Bosch GmbH
70442 Stuttgart (DE)**

(72) Erfinder:
- **VIANELLO, Alessandro
  71272 Renningen (DE)**
- **ANGST, Marcel
  70563 Stuttgart (DE)**
- **LOUISON, Bastien
  400000 Cluj-Napoca (RO)**

(56) Entgegenhaltungen:
EP-A1- 1 475 633  DE-A1- 102009 043 001
DE-A1- 19 625 763  DE-A1- 2 753 635
GB-A- 2 521 767  US-A- 3 766 387
US-A1- 2006 114 002

**Beschreibung**

Die vorliegende Erfindung betrifft ein Verfahren zur Detektion von Inhomogenitäten, insbesondere von nichtmetallischen Einschlüssen, in einem Werkstück.

Stand der Technik

Die Festigkeit eines metallischen Bauteils hängt wesentlich vom Reinheitsgrad des Werkstoffs ab. Nichtmetallische Einschlüsse oder andere Fehlstellen sind Schwachstellen, an denen der Werkstoff bei hoher Beanspruchung des Bauteils bevorzugt versagen kann. Daher besteht ein Bedarf, in der Produktion den Reinheitsgrad des Werkstoffs zu überwachen.

Durch mikroskopische Untersuchung metallographischer Schliffe können Fehlstellen mit einer Ortsauflösung erfasst werden, die der Auflösung des verwendeten Mikroskops entspricht. Da eine entsprechende Präparation der Probe erforderlich ist, ist die Untersuchung vergleichsweise zeitaufwändig.

Daher wird als gängiges Verfahren für die Prüfung metallischer Werkstoffe die Ultraschall-Tauchtechnik angewendet. Hierbei wird ein Prüfkörper aus dem Werkstoff in einer Ankopplungsflüssigkeit, beispielsweise Wasser, mit Ultraschallwellen durchstrahlt. An den Grenzflächen zu Fehlstellen werden die Ultraschallwellen teilweise reflektiert. Ein Quasi-Standard für dieses Prüfverfahren ist im Stahl-Eisen-Prüfblatt SEP 1927, welches beim Stahl-Eisen-Verlag erhältlich ist, beschrieben. Entsprechende Prüfvorrichtungen sind beispielsweise aus der DE 40 36 005 A1, aus der EP 1 475 633 A1, aus der US 6 318 178 B1 sowie aus der WO 2006 120 875 A1 bekannt.

Aus der DE 196 25 763 A1 ist eine Vorrichtung zur Qualitätsprüfung von Reifen bekannt, bei der ein Reifen einem Feld ausgesetzt wird. Eine Auswerteeinrichtung detektiert eine Feldänderung, die ein Maß für den Zustand des Reifens ist.

Aus der DE 10 2009 043 001 A1 ist ein Verfahren zur Bestimmung von Defekten in einem für elektromagnetische Wellen transparenten Material, insbesondere für optische Zwecke, bekannt.

Aus der US 3,766,387 ist eine zerstörungsfreie Testvorrichtung bekannt, die mittels Strahlung Materialdefekte detektiert.

Aus der GB 2521767 A ist ein Abtastverfahren bekannt, das mittels Gammastrahlung und Strahlungsdetektoren Defekte in Unterwasserpipelines detektiert.

Es hat sich in den Versuchen der Anmelderin gezeigt, dass auch nichtmetallische Einschlüsse, die zu klein sind, um mit der derzeitigen Ultraschall-Tauchtechnik verlässlich erkannt werden zu können, die Festigkeit des Werkstoffs entscheidend schwächen und zu Bauteilausfällen führen können.

Offenbarung der Erfindung

Im Rahmen der Erfindung wurde ein Verfahren zur Detektion eines Markers, der auf eine Inhomogenität im Material eines Werkstücks hindeutet, entwickelt. Dieser Marker wird aus einem Signal, mit dem das Werkstück einem Empfänger auf eine Abfragestrahlung aus einem Sender antwortet und/oder geantwortet hat, ausgewertet. Die Abfragestrahlung kann insbesondere Schallwellen, elektromagnetischer Strahlung und/oder ionisierender Strahlung umfassen.

Die Perfektform "geantwortet hat" ist dahingehend zu verstehen, dass zur Auswertung nicht nur "frische" Messdaten herangezogen werden können, die eigens für die Durchführung des Verfahrens aufgenommen wurden, sondern auch bereits existierende Messdaten mit dem Verfahren neu ausgewertet werden können. Dies ist insbesondere dann vorteilhaft, wenn das Werkstück nicht mehr für die Aufnahme weiterer Messdaten zur Verfügung steht, beispielsweise, weil es zwischenzeitlich einer zerstörenden Untersuchung unterzogen wurde.

Der Begriff des Werkstücks ist nicht auf Prüfkörper beschränkt, die eigens für die Materialprüfung hergestellt wurden. Das Werkstück kann auch ein Bauteil oder ein Vorprodukt, dessen Weiterverarbeitung zu einem Bauteil vorgesehen ist, sein.

Erfindungsgemäß wird der Marker aus einem Anteil des Signals ausgewertet, , der sich bei einer Drehung und/oder Verschwenkung des Werkstücks, und/oder des Senders, und/oder des Empfängers, um einen Winkel $\varphi$ mit dem Winkel $\varphi$ periodisch ändert.

Hierunter ist zu verstehen, dass aus dem Signal zunächst derjenige Anteil absepariert wird, der sich mit dem Winkel $\varphi$ periodisch ändert, und dass anschließend aus diesem Anteil der Marker ausgewertet wird.

Dieses Absepariaren kann beispielsweise offline nach der vollständigen Erfassung der Messdaten erfolgen. Es kann aber auch zur weiteren Verbesserung der Empfindlichkeit beispielsweise während der Datenaufnahme eine schnelle Drehung oder Verschwenkung mit einer vorgegebenen Kreisfrequenz $\omega$ erfolgen und, beispielsweise mit einem Lock-In-Verstärker, bevorzugt ein Signal detektiert werden, das sich mit dieser Kreisfrequenz $\omega$ ändert.

Es wurde erkannt, dass durch die Auswertung des vom Winkel $\varphi$ abhängigen Signalanteils Signale, die von tatsächlichen Fehlstellen im Werkstück ausgehen, deutlich schärfer von Hintergrundrauschen und Scheindetektionen getrennt werden können als dies nach dem bisherigen Stand der Technik möglich war. Speziell bei der Untersuchung mit der Ultraschall-Tauchtechnik kann die Empfindlichkeit erheblich verbessert werden, so dass deutlich kleinere Fehlstellen verlässlich erkannt werden können.

Die Erfindung geht von dem Grundgedanken aus, dass das Werkstück nicht mehr oder weniger homogen von Fehlstellen durchsetzt ist, sondern dass derartige Fehlstellen sporadisch auftreten. Innerhalb des festen Werkstücks ist eine Fehlstelle jeweils ortsfest. Wird nun das Werkstück um den Winkel φ gedreht oder verschwenkt, so ändert sich die relative Geometrie zwischen dem Sender, der Fehlstelle und dem Werkstück. Diese Änderung wirkt sich auf das registrierte Signal aus, das somit eine Komponente erhält, die mit einer wie auch immer gearteten stetigen Funktion vom Winkel φ abhängt. Die Quellen des Hintergrundrauschens hingegen sind im Werkstück in Lage und Orientierung statistisch verteilt und zeigen daher im Mittel keine funktionale Abhängigkeit vom Winkel φ.

In einer besonders vorteilhaften Ausgestaltung der Erfindung wird ein zylindrisches Werkstück gewählt, und die Drehung erfolgt um die Zylinderachse des Werkstücks, bzw. sie ist bei der Aufnahme des Signals in dieser Weise erfolgt. Wird nun beispielsweise die Abfragestrahlung an einem vorgegeben Ort in die Mantelfläche des Zylinders eingekoppelt und das Signal am gleichen Ort oder an einem anderen Ort aus der Mantelfläche des Zylinders in Richtung auf den Empfänger ausgekoppelt, so sollte sich das Signal bei einem vollständig homogenen Werkstück infolge der Drehung nicht ändern. Ein Anteil des Signals, der mit dem Winkel φ der Drehung korreliert ist, rührt mit hoher Wahrscheinlichkeit von einer Inhomogenität im Material des Werkstücks her.

In einer weiteren besonders vorteilhaften Ausgestaltung der Erfindung wird eine Abfragestrahlung gewählt, die an der Inhomogenität reflektiert wird und die vom Material des Werkstücks nur so weit abgeschwächt wird, dass das reflektierte Signal noch vom Empfänger detektiert werden kann. Hierfür eignet sich insbesondere Ultraschall. An den Grenzflächen zu Fehlstellen ändert sich die lokale Schallgeschwindigkeit und somit auch der Brechungsindex für die Abfragestrahlung, so dass die Abfragestrahlung teilweise reflektiert wird. Die Schallgeschwindigkeit liegt in einem Bereich, in dem es technisch vergleichsweise einfach ist, aus der Laufzeit T des empfangenen Signals auf die Tiefe zurückzuschließen, in der der Ursprungsort des Signals im Werkstück, d.h. die Fehlstelle, liegt. Das Prinzip bleibt unverändert, wenn eine elektromagnetische Abfragestrahlung gewählt wird, jedoch ist die Messung der Laufzeit T aufwändiger, da die Lichtgeschwindigkeit deutlich höher als die Schallgeschwindigkeit ist. Ist die Abfragestrahlung eine ionisierende Strahlung, die nach einem statistischen Zeitprogramm emittiert wird, kann für die Messung der Laufzeit beispielsweise eine Koinzidenzschaltung verwendet werden.

In einer besonders vorteilhaften Ausgestaltung der Erfindung wird der mit dem Winkel φ veränderliche Anteil des Signals aus einer Darstellung des Signals ausgewertet, in der eine Amplitude A des Signals als Funktion des Winkels φ einerseits sowie einer Laufzeit T des Signals, und/oder einer aus der Laufzeit T des Signals abgeleiteten Größe q, andererseits aufgetragen ist.

Die Laufzeit T ist insbesondere ein Maß dafür, welche Strecke die Abfragestrahlung vom Sender bis zu einer Fehlstelle innerhalb des Materials des Werkstücks zurücklegt und welche Strecke das an der Fehlstelle erzeugte Signal auf dem Weg zum Empfänger innerhalb des Materials des Werkstücks zurücklegt. Diese Strecke hängt von der relativen Geometrie zwischen dem Sender, dem Empfänger und der Fehlstelle ab. In der genannten Auftragung über dem Winkel φ einerseits und der Laufzeit T, bzw. der abgeleiteten Größe q, andererseits sollte eine echte Fehlstelle im Werkstück somit zu besonders hohen Amplituden A des Signals bei Werten von T bzw. q führen, die mit dem Winkel φ periodisch sind. Auf diese Weise lässt sich eine echte Fehlstelle nicht nur von Hintergrundrauschen, sondern auch von anderweitigen Scheindetektionen sicher unterscheiden.

Besonders vorteilhaft wird der Abstand X zwischen dem Ursprungsort des Signals im Werkstück und einem vorgegebenen Ort P, an dem das Signal aus dem Werkstück austritt oder ausgetreten ist, als abgeleitete Größe q gewählt. Dieser Abstand X ist eine Näherung für die von der Abfragestrahlung, bzw. vom Signal, im Material des Werkstücks zurückzulegende Strecke mit einer vergleichsweise einfachen funktionalen Abhängigkeit vom Winkel φ.

Um den mit dem Winkel φ veränderlichen Anteil des Signals abzuseparieren, wird in einer besonders vorteilhaften Ausgestaltung der Erfindung der Verlauf der Amplitude A des Signals entlang mindestens einer Bahnkurve, die die T- bzw. q-Koordinate eines festen Ursprungsorts des Signals in Abhängigkeit der φ-Koordinate dieses Ursprungsorts beschreibt, ermittelt.

Die relative Geometrie zwischen der Fehlstelle, dem Sender und dem Empfänger hängt insbesondere von der Position der Fehlstelle innerhalb des Werkstücks ab. Ist beispielsweise das Werkstück ein Zylinder und wird ein näherungsweise scheibenförmiges Teilvolumen dieses Zylinders untersucht, so sind die maßgeblichen Freiheitsgrade für die Position der Fehlstelle der radiale Abstand vom Zentrum der Scheibe und die azimutale Winkelposition auf der Scheibe. Diese beiden Größen beeinflussen beispielsweise die funktionale Abhängigkeit des Abstandes X vom Winkel φ. Da die Fehlstelle genau das ist, was mit dem Verfahren überhaupt erst detektiert werden soll, ist die Position der Fehlstelle nicht bekannt. Um nun die Existenz der Fehlstelle zu detektieren und zugleich auch eine Aussage über ihre Position zu erhalten, wird in einer weiteren besonders vorteilhaften Ausgestaltung der Erfindung für die Bahnkurve ein Ansatz mit einem Satz freier Parameter aufgestellt, wie beispielsweise die beiden Freiheitsgrade für die Position der Fehlstelle. Die Ermittlung des Verlaufs der Amplitude A des Signals wird nun für eine Vielzahl von Wertevektoren der Parameter wiederholt wird. Stimmt für einen dieser Wertevektoren der Verlauf der Amplitude A des Signals besonders plausibel mit der Hypothese überein, dass eine Fehlstelle im Werkstück vorhanden ist, so ist zugleich plausibel, dass der Wertevektor die der Realität entsprechenden Werte für die freien Parameter enthält. Somit liegen in dem genannten Beispiel die Polarkoordinaten der Fehlstelle innerhalb des scheibenförmigen untersuchten Teilvolumens fest.

In einer weiteren besonders vorteilhaften Ausgestaltung der Erfindung wird für jeden Wertevektor der Parameter die Summe $G_{sum}$ der Amplituden A des Signals entlang der zugehörigen Bahnkurve in der Darstellung des Signals ermittelt. Auf diese Weise lässt sich quantifizieren, zu welchem Grad der Verlauf der Amplitude A des Signals mit der Hypothese übereinstimmt, dass eine Fehlstelle vorhanden ist und zugleich der Wertevektor die der Realität entsprechenden Werte für die freien Parameter enthält.

Für eine belastbare Aussage, dass eine Fehlstelle im Werkstück vorhanden ist, sollte sich der periodisch vom Winkel φ abhängige Verlauf der Amplitude A mit einem gewissen Mindestmaß an Kontrast vom Hintergrundrauschen abheben. Daher wird in einer weiteren besonders vorteilhaften Ausgestaltung der Erfindung derjenige Wertevektor, für die die Summe $G_{sum}$ ihr Maximum $G_{max}$ annimmt, als Marker oder als Kandidat für einen Marker gewertet, wenn das Verhältnis des Maximums $G_{max}$ zum Mittelwert oder Median $G_{mean}$ aller Amplituden A in der Darstellung des Signals einen vorgegebenen Schwellwert S erreicht oder überschreitet.

So kann beispielsweise bereits aus der Untersuchung eines scheibenförmigen Teilvolumens eines zylindrischen Werkstücks geschlossen werden, dass das Werkstück eine Fehlstelle enthält. Diese Feststellung kann aber auch vorteilhaft mit der Untersuchung eines weiteren Teilvolumens des Werkstücks plausibilisiert werden. Daher werden in einer weiteren besonders vorteilhaften Ausgestaltung der Erfindung mindestens zwei über der Laufzeit T bzw. der abgeleiteten Größe q einerseits und über dem Winkel φ andererseits aufgetragene Darstellungen von Signalen, die sich auf benachbarte und/oder überlappende Teilvolumina des Werkstücks beziehen, ausgewertet. Es kann dann vorteilhaft insbesondere auf einen Marker für eine Fehlstelle geschlossen werden, wenn die aus den zu Neue Seite 7 der Beschreibung beiden Teilvolumina gehörigen Darstellungen jeweils ermittelten Kandidaten innerhalb eines vorgegebenen Abstands D zueinander liegen.

Auf diese Weise können zusätzlich beispielsweise die Größe, die Ausdehnung und der Verlauf der Fehlstelle in dem Werkstück in allen drei Raumdimensionen bestimmt werden. Beispielsweise kann die Fehlstelle in einem ersten scheibenförmigen Teilvolumen im Zentrum beginnen und in den entlang der Zylinderachse daran anschließenden Teilvolumina immer weiter in den linken oberen Quadranten der Scheibe wandern. Diese genauere Kenntnis kann beispielsweise für die Ursachenforschung in Bezug auf die Herkunft der Fehlstelle genutzt werden.

Die Erkennung eines vom Winkel φ abhängigen Signalanteils ist nicht auf die hier beschriebene Methode beschränkt. Jede geeignete Methode zur Mustererkennung kann verwendet werden. Insbesondere kann das Signal hierzu in einen anderen mathematischen Raum transformiert werden, in dem der vom Winkel φ abhängige, und/oder im Winkel φ periodische, Anteil des Signals klarer hervortritt. Beispielsweise ist die Hough-Transformation besonders geeignet, um Geraden, Kreise oder beliebige andere parametrisierbare geometrische Figuren in Schwarz-Weiß-Bildern zu erkennen. Die Fourier-Transformation hebt speziell periodische Signalanteile hervor. Es kann aber auch beispielsweise ein neuronales Netz auf die Erkennung eines periodischen Signalanteils trainiert werden.

Mit dem Verfahren lässt sich eine Vorrichtung für die zerstörungsfreie Materialprüfung von Werkstücken dahingehend aufwerten, dass kleinere Inhomogenitäten erkannt werden als dies bislang möglich war. Die Erfindung ist so, wie sie in den Ansprüchen definiert ist.

Erfindungsgemäß sind Mittel vorgesehen, die dazu ausgebildet sind, ein Verfahren gemäß der Erfindung auszuführen.

Das Verfahren kann auch an einer unveränderten Ultraschall-Prüfanlage durchgeführt werden. Nach dem zuvor Gesagten können auch bereits vorliegende Daten neu ausgewertet werden, so dass neue Erkenntnisse über Inhomogenitäten im Material eines Werkstücks gewonnen werden können, ohne das Werkstück ein weiteres Mal in die Hand zu nehmen. Somit ist das Verfahren insbesondere teilweise oder gar vollständig in Software implementierbar, und diese Software ist ein eigenständig verkaufbares Produkt, dessen Benutzung das Vorhandensein einer physischen Prüfanlage nicht voraussetzt. Die Erfindung bezieht sich daher auch auf ein Computerprogrammprodukt mit maschinenlesbaren Anweisungen, die, wenn sie auf einem Computer ausgeführt werden, den Computer dazu veranlassen, ein Verfahren gemäß der Erfindung auszuführen.

Hauptanwendungsfeld der Erfindung ist die Qualitätskontrolle von Stählen, aus denen besonders stark belastete und/oder besonders sicherheitsrelevante Bauteile für Fahrzeuge hergestellt werden sollen, auf mesoskopische nichtmetallische Einschlüsse. Beispiele für derartige Bauteile sind Injektoren für Diesel-Einspritzsysteme, Bauteile für Hochdruckpumpen in Diesel-Einspritzsystemen, Bauteile im Antriebsstrang derartiger Hochdruckpumpen sowie Achsen und andere Fahrwerksteile. An Injektoren sind beispielsweise die Düse und die Hochdruckbohrung besonders hoch belastet.

In den Versuchen der Anmelderin konnten in Probenmaterial aus Vergütungsstahl mit der Ultraschall-Tauchtechnik gemäß Prüfblatt SEP 1927 drei nichtmetallische Einschlüsse mit einer minimalen Größe KSR oberhalb von 0,3 mm erkannt werden, während mit dem Verfahren gemäß der Erfindung in dem gleichen Werkstück 45 nichtmetallische Einschlüsse mit einer minimalen Größe KSR oberhalb von 0,2 mm erkannt wurden. Wäre der Stahl zu hochbelasteten Bauteilen verarbeitet worden, hätten diese Einschlüsse zu einem frühzeitigen Ausfall der Bauteile führen können.

Weitere, die Erfindung verbessernde Maßnahmen werden nachstehend gemeinsam mit der Beschreibung der bevorzugten Ausführungsbeispiele der Erfindung anhand von Figuren näher dargestellt.

Ausführungsbeispiele

Es zeigt:

Figur 1 Grundprinzip der Ultraschall-Tauchtechnik zur Untersuchung eines Werkstücks 1;

Figur 2 Extraktion des von einer Inhomogenität 2a im Material 2 des Werkstücks 1 herrührenden Signals aus dem gemessenen Signal 6;

Figur 3 Erkennung des im Winkel $\varphi$ periodischen Anteils 6a des Signals 6 mit einem parametrisierten Ansatz 6e für Bahnkurven 6d, 6d';

Figur 4 Zusammenfassung des Verfahrens 100 einschließlich Plausibilisierung 126 mehrerer Kandidaten 7a, 7a' für einen Marker 7, der auf eine Inhomogenität 2a im Material 2 des Werkstücks 1 hindeutet.

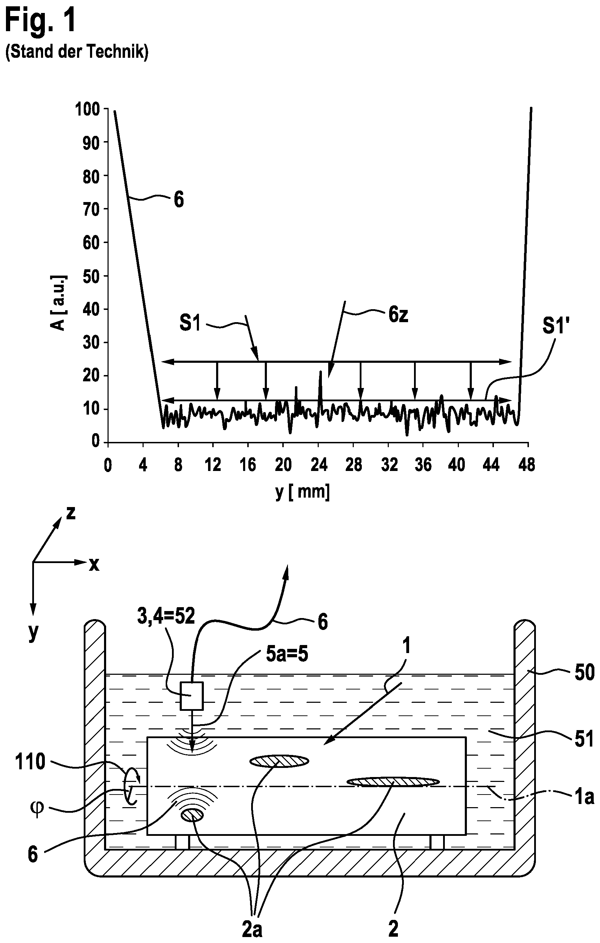

Nach der nicht maßstabsgetreuen Figur 1 werden in einem Tauchbecken 50, das mit Wasser 51 als Ankopplungsmedium gefüllt ist, Ultraschallwellen 5a als Abfragestrahlung 5 von dem Sender 3 in das Werkstück 1 gestrahlt. Dort, wo das Material 2 des Werkstücks 1 nichtmetallische Einschlüsse 2a aufweist, ruft die Abfragestrahlung 5 ein Echosignal 6 hervor, das von dem Empfänger 4 aufgefangen wird.

Der Prüfkopf 52 mit dem Sender 3 und dem Empfänger 4 ist in der x-Richtung verfahrbar. Zugleich ist das zylindrische Werkstück 1 um seine Zylinderachse 1a drehbar 110. Für jede x-Position des Prüfkopfes 52 und für jede Winkelposition $\varphi$ des Werkstücks 1 lässt sich die Amplitude A des Signals 6 als Funktion der y-Koordinate ermitteln, von der das reflektierte Signal 6 herrührt. Die y-Koordinate ergibt sich jeweils aus der Laufzeit T des Signals 6. Die Auftragung von A über y, die in Figur 1 beispielhaft für einen Wert der axialen Position x des Prüfkopfes 52 und einen Wert des Winkels $\varphi$ im Diagramm dargestellt ist, wird als A-Scan bezeichnet.

Die sehr starken Echos am linken und am rechten Rand des Diagramms rühren von Reflexionen an der Grenzfläche zwischen dem Werkstück 1 und dem Wasser 51 her. Die Inhomogenität 2a im Material 2 des Werkstücks 1 ruft einen Peak 6z im Signal 6 hervor. Dieser Peak 6z liegt im Bereich des Hintergrundrauschens, so dass aus ihm nicht verlässlich auf das Vorhandensein der Inhomogenität 2a geschlossen werden kann.

Nach dem bisherigen Stand der Technik wurde nur das Maximum der Amplitude A weiter ausgewertet, sofern es über einer Detektionsschwelle S1 lag. In Abhängigkeit von y und $\varphi$ wurde dieses Maximum in einem C-Scan genannten Diagramm aufgetragen. Hieraus wurde eine kumulierte Länge von Fehlstellen entlang der y-Achse ermittelt. Das Verhältnis dieser kumulierten Länge zum Prüfvolumen war der Fehlerindex, der als Maß für die Reinheit des Materials 2 des Werkstücks 1 herangezogen wurde.

Der Wert der Detektionsschwelle S1 ist in Figur 1 eingezeichnet. Die kleine Inhomogenität 2a, die in der in Figur 1 gezeigten Situation untersucht wird, wird mit der bisherigen Auswertungsmethode nicht als solche erkannt. Eine Senkung der Detektionsschwelle S1 auf einen Wert S1' ist ebenfalls keine Lösung, da sich dann Fehldetektionen häufen. Der (in Figur 1 nicht gezeigte) C-Scan wird hierdurch zu einer ausgefüllten Fläche, die nicht mehr aussagefähig ist.

In dem in Figur 1 gezeigten Beispiel hat der Prüfkopf 52 einen Durchmesser von 6 mm und sendet mit einer Frequenz von 10 MHz. Zur Kalibrierung der Skala für die Amplitude wird ein Vergleichskörper untersucht, der eine 1 mm tiefe Bohrung in seiner Frontseite aufweist. Die Verstärkung wird so eingepegelt, dass diese Bohrung zu einer Amplitude von 80 % des Maximums führt und dass die Absorption der Abfragestrahlung 5 durch das Material 2 ausgeglichen wird. Mit der Auswertung gemäß Stand der Technik können nichtmetallische Einschlüsse 2a bis hinab zu einer minimalen Größe von 0,3 mm erkannt werden. Die Ortsauflösung beträgt 1 mm in x-Richtung und 0,25 mm in Umfangsrichtung des Werkstücks 1.

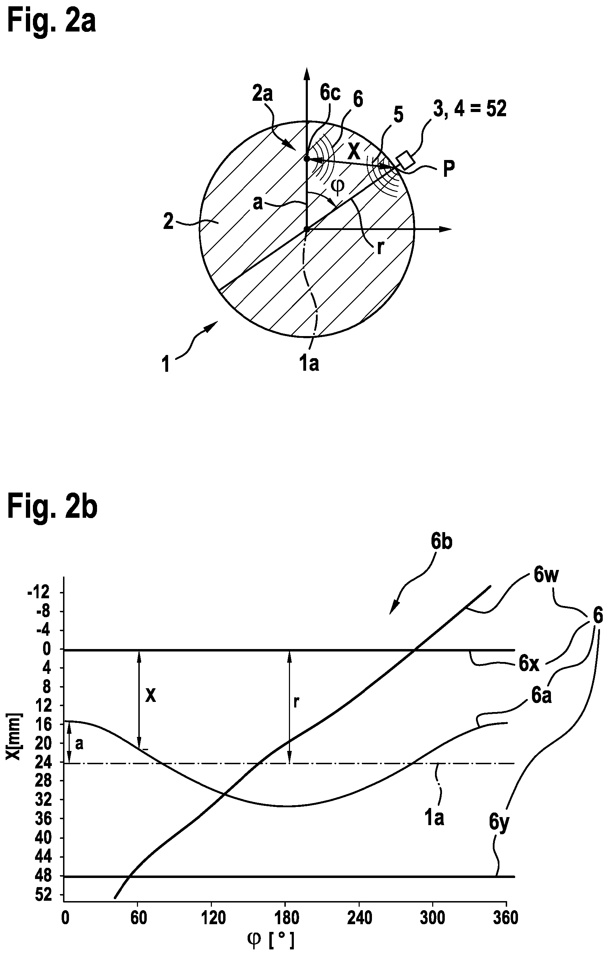

Figur 2 verdeutlicht an einem beispielhaften zylindrischen Werkstück 1 mit Radius r, dessen Material 2 nur eine einzige Inhomogenität 2a in einem Abstand a zur Zylinderachse 1a des Werkstücks 1 und in einer Winkelposition $\varphi$ zur Verbindungslinie zwischen Zylinderachse 1a und Prüfkopf 52 enthält, die Abhängigkeit des von dieser Inhomogenität 2a herrührenden Signals 6 vom Drehwinkel $\varphi$ des Werkstücks 1 um die Zylinderachse 1a.

Figur 2a ist eine Schnittzeichnung an der axialen Position x, an der sich der Prüfkopf 52 befindet, und erläutert insbesondere die Ermittlung des Abstandes X zwischen dem Ursprungsort 6c des Signals 6 im Werkstück 1 und dem Ort P, an dem das Signal 6 aus dem Werkstück 1 austritt. Der Abstand X ist gegeben durch

$$X = \sqrt{a^2 + r^2 - 2 \cdot a \cdot r \cdot \sin(\varphi + \varphi_0)} \qquad (1)$$

mit einer Phasenverschiebung $\varphi_0$.

Figur 2b zeigt eine Darstellung 6b, in der die Amplitude des Signals 6 über dem Abstand X und dem Winkel $\varphi$ aufgetragen ist. Das Signal 6 hat neben dem Anteil 6a, der sich in Abhängigkeit des Winkels $\varphi$ ändert, noch einen Anteil 6x, der von der Reflexion an der Grenzfläche vom Wasser 51 zum Werkstück 1 herrührt, sowie einen Anteil 6y, der von der Reflexion an der gegenüberliegenden Grenzfläche vom Werkstück 1 zum Wasser 51 herrührt. Die Anteile 6x und 6y sind vom Winkel $\varphi$ unabhängig. Ein weiterer Signalanteil 6w, der von einer Scheindetektion herrührt, hängt zwar vom Winkel $\varphi$ ab, aber nicht in periodischer Weise.

Somit lässt sich der Anteil 6a beispielsweise aus dem Signal 6 abseparieren, indem in einer Darstellung 6b, wie sie in Figur 2b gezeigt ist, nach einem im Winkel $\varphi$ periodischen Signal gesucht wird. Hierfür kann jede beliebige Methode zur Mustererkennung verwendet werden.

Die Darstellung 6b kann beispielsweise als Grauwertdatenmatrix generiert und visualisiert werden. Ein 8-Bit-Grauwert G kann dann aus der zugehörigen Amplitude A beispielsweise mit der Skalierung

$$G = 255 - A \cdot \frac{255}{100\,\%} \qquad (2)$$

ermittelt werden. Das Grauwertbild kann in beliebiger Weise softwaremäßig kontrastverstärkt werden, um eine höhere Prüfempfindlichkeit zu erreichen.

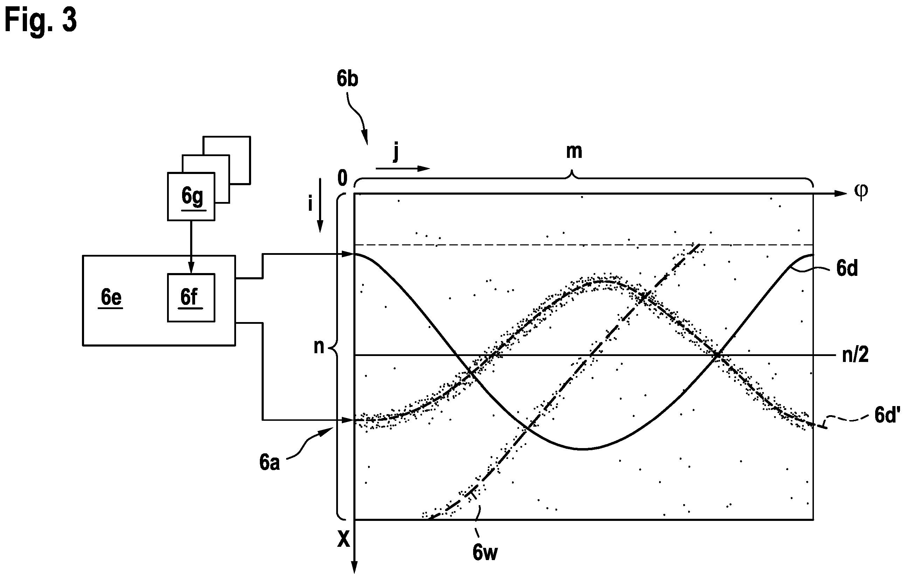

Figur 3 zeigt schematisch ein derartiges Grauwertbild, welches in der $\varphi$-Koordinate m=360 Pixel und in der X-Koordinate n=512 Pixel umfasst, als Darstellung 6b des Signals 6. Ein im Winkel $\varphi$ periodischer Anteil 6a des Signals 6 sowie ein in nicht periodischer Weise vom Winkel $\varphi$ abhängiger Anteil 6w des Signals 6, der von einer Scheindetektion herrührt, heben sich so klar vom Hintergrundrauschen ab, dass sie mit bloßem Auge erkennbar sind. Figur 3 erläutert auch die automatische Erkennung des periodischen Anteils 6a des Signals 6 in der Darstellung 6b.

Hierzu wird Gleichung (1) zu einer Koordinatengleichung 6e umgeformt, die für alle Bahnkurven 6d jeweils die Koordinaten (i,j) der zur Bahnkurve 6d gehörigen Pixel miteinander verknüpft:

$$i = \mathrm{round}\left(\sqrt{a^2 + \left(\frac{n}{2}\right)^2 - 2 \cdot a \cdot \left(\frac{n}{2}\right) \cdot \sin(j + \varphi_0)}\right) \qquad (3)$$

Hierin ist n für alle Bahnkurven 6d gleich, während der radiale Abstand a der Inhomogenität 2a von der Zylinderachse sowie die Phasenverschiebung $\varphi_0$ einen Satz 6f freier Parameter bilden. Es werden nun alle möglichen diskreten Werte a=[0,...,n/2] für a und $\varphi_0$=[0,...,360°] für $\varphi_0$ abgerastert, und für jeden möglichen Wertevektoren 6g=(a,($\varphi_0$) wird die zugehörige Bahnkurve 6d bestimmt.

Entlang jeder Bahnkurve 6d werden nun für alle zur Bahnkurve 6d gehörigen Pixel mit Koordinaten (i,j) die Grauwerte G(i,j) bestimmt und zur Summe

$$G_{sum}(a, \varphi_0) = \sum_{i,j} G(i,j) \,|\, (i,j) \in 6\mathrm{d}(a, \varphi_0) \qquad (4)$$

aufsummiert.

Die Übereinstimmung der in Figur 3 beispielhaft gezeigten Bahnkurve 6d mit dem periodischen Anteil 6a des Signals 6 ist noch nicht besonders gut. Die Bahnkurve 6d' hingegen, die für einen den Wertevektor 6g=(a$_1$,$\varphi_{0,1}$) erstellt wurde, bildet exakt den Verlauf des periodischen Anteils 6a des Signals 6 nach. Dementsprechend nimmt auch die Summe $G_{sum}(a,\varphi_0)$ für diesen Wertevektor ihr Maximum $G_{max}$ an.

Im vorliegenden Beispiel ist das Verhältnis dieses Maximums $G_{max}$ zum Mittelwert $G_{mean}$ aller Grauwerte in der Darstellung 6b größer als der vorgegebene Schwellwert S, so dass der Wertevektor 6g=(a$_1$,$\varphi_{0,1}$) als Kandidat 7a für einen Marker 7 gewertet wird.

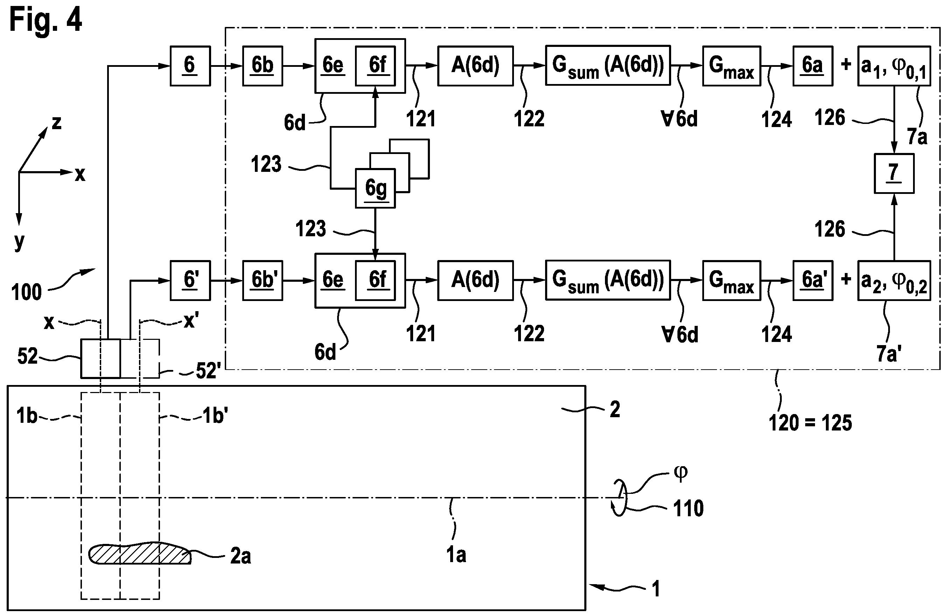

Figur 4 fasst das Verfahren 100 für die Signalauswertung noch einmal schematisch zusammen und erläutert zugleich, wie der in der beschriebenen Weise ermittelte Kandidat 7a für den Marker 7 weiter plausibilisiert werden kann.

Wenn der Prüfkopf 52 in einer ersten Position x ist, wird ein erstes scheibenförmiges Teilvolumen 1b des zylindrischen Werkstücks 1 untersucht. Das Werkstück 1 wird einmal komplett um seine Achse 1a gedreht 110, und das Signal 6 wird aufgenommen.

Wenn der Prüfkopf 52 in einer zweiten Position x' ist, wird ein zweites scheibenförmiges Teilvolumen 1b' des zylindrischen Werkstücks 1 untersucht. Der Prüfkopf 52 ist in dieser Position x' gestrichelt eingezeichnet mit dem Bezugszeichen 52'. Das Werkstück 1 wird einmal komplett um seine Achse 1a gedreht 110, und das Signal 6' wird aufgenommen.

Die Signale 6 und 6' werden nun in Block 120=125 weiter ausgewertet.

Aus dem Signal 6 bzw. 6' wird die Darstellung 6b bzw. 6b' erhalten, die die Amplitude A des Signals 6 bzw. 6' in Abhängigkeit von X und dem Drehwinkel $\varphi$ angibt. Es wird nun in Schritt 123 ein parametrisierter Ansatz 6e mit freien Parametern 6f für Bahnkurven 6d von in dem Drehwinkel $\varphi$ periodischen Funktionen aufgestellt, und durch Einsetzen aller möglichen Wertevektoren 6g für die freien Parameter 6f werden alle möglichen Bahnkurven 6d ermittelt.

Entlang jeder dieser Bahnkurven 6d wird in Schritt 121 der Verlauf der Amplitude A des Signals 6 bzw. 6' ermittelt. Dieser Verlauf wird in Schritt 122 jeweils zur Grauwertsumme $G_{sum}$ kondensiert, die einem bestimmten Verlauf der Amplitude und somit auch einer bestimmten Bahnkurve 6d, zugeordnet ist. Das über alle Bahnkurven 6d gebildete Maximum $G_{max}$ der Grauwertsummen $G_{sum}$ ist derjenigen Bahnkurve 6d zugeordnet, die in bester Übereinstimmung mit dem periodischen Anteil 6a bzw. 6a' des Signals 6 bzw. 6' ist. Diese Bahnkurve 6d gibt somit den periodischen Anteil 6a bzw. 6a' des Signals 6 bzw. 6' an. Die Werte $a_1$ und $\varphi_{0,1}$, bzw. $a_2$ und $\varphi_{0,2}$, durch deren Einsetzen als freie Parameter 6f die Bahnkurve 6d erhalten wurde, werden in Schritt 124 als Kandidaten 7a bzw. 7a' für den auf die Inhomogenität 2a im Material 2 des Werkstücks hindeutenden Marker 7 gewertet.

Beide Kandidaten 7a und 7a' werden in Schritt 126 gegeneinander plausibilisiert. Hierzu wird geprüft, ob die beiden Kandidaten 7a und 7a' für den Marker 7 innerhalb eines vorgegebenen Abstandes D zueinander liegen, ob also in beiden aneinander angrenzenden Teilvolumina 1b und 1b' des zylindrischen Werkstücks 1 die Inhomogenität 2a letztlich an der gleichen Stelle im Material 2 des Werkstücks 1 detektiert wird. In dem in Figur 4 gezeigten Beispiel wird geprüft, ob sowohl $|a_1-a_2| < \Delta a$ als auch $\varphi_{0,1}-\varphi_{0,2} < \Delta\varphi$ erfüllt sind, mit voneinander unabhängigen Schwellwerten $\Delta a$ und $\Delta\varphi$. In dem in Figur 4 gezeigten Beispiel sind beide Bedingungen erfüllt, und es wird auf einen Marker 7 für das Vorliegen der Inhomogenität 2a im Material 2 des Werkstücks 1 geschlossen.

Wenn in der aus dem Signal 6' ermittelten Darstellung 6b' kein im Winkel $\varphi$ periodischer Anteil 6a' erkannt wird, so kann auch keine Bahnkurve 6d in Übereinstimmung mit einem solchen periodischen Anteil 6a' in Übereinstimmung gebracht werden. Es gibt dann weder ein $a_2$ noch ein $\varphi_{0,2}$, so dass die Plausibilisierung negativ ausfällt.

In gleicher Weise lässt sich das Verfahren für weitere Teilvolumina des Werkstücks 1 fortsetzen, so dass am Ende eine Ergebnismatrix entsteht, in der alle Inhomogenitäten 2a des Materials des Werkstücks 1 in Tiefenlage, Winkel und axialer Position kartographiert sind.

**Patentansprüche**

1. Verfahren (100) zur Detektion eines Markers (7), der auf eine Inhomogenität (2a) im Material (2) eines Werkstücks (1) hindeutet, in einem Signal (6), mit dem das Werkstück (1) einem Empfänger (4) auf eine Abfragestrahlung (5) aus einem Sender (3) antwortet und/oder geantwortet hat,

   **dadurch gekennzeichnet, dass** aus dem Signal (6) zunächst ein in einem Winkel $\varphi$ periodischer Anteil (6a) absepariert wird, der sich bei einer Drehung und/oder Verschwenkung (110) des Werkstücks (1), und/oder des Senders (3),
   und/oder des Empfängers (4), um den Winkel $\varphi$ mit dem Winkel $\varphi$ periodisch ändert, und dass anschließend aus diesem Anteil (6a) der Marker (7) ausgewertet wird (120).

2. Verfahren (100) nach Anspruch 1, **dadurch gekennzeichnet, dass** ein zylindrisches Werkstück (1) gewählt wird und die Drehung (110) um die Zylinderachse (1a) des Werkstücks (1) erfolgt oder erfolgt ist.

3. Verfahren (100) nach einem der Ansprüche 1 bis 2, **dadurch gekennzeichnet, dass** eine Abfragestrahlung (5) gewählt wird, die an der Inhomogenität (2a) reflektiert wird und die vom Material (2) des Werkstücks (1) nur so weit abgeschwächt wird, dass das reflektierte Signal (6) noch vom Empfänger (4) detektiert werden kann.

4. Verfahren (100) nach einem der Ansprüche 1 bis 3, **dadurch gekennzeichnet, dass** der mit dem Winkel $\varphi$ veränderliche Anteil (6a) des Signals (6) aus einer Darstellung (6b) des Signals (6) ausgewertet wird (120), in der eine Amplitude A des Signals (6) als Funktion des Winkels $\varphi$ einerseits sowie einer Laufzeit T des Signals (6), und/oder einer aus der Laufzeit T des Signals (6) abgeleiteten Größe q, andererseits aufgetragen ist.

5. Verfahren (100) nach Anspruch 4, **dadurch gekennzeichnet, dass** der Abstand X zwischen dem Ursprungsort (6c) des Signals (6) im Werkstück (1) und einem vorgegebenen Ort P, an dem das Signal (6) aus dem Werkstück (1) austritt oder ausgetreten ist, als abgeleitete Größe q gewählt wird.

6.  Verfahren (100) nach einem der Ansprüche 4 bis 5, **dadurch gekennzeichnet, dass** der Verlauf der Amplitude A des Signals (6) entlang mindestens einer Bahnkurve (6d), die die T- bzw. q-Koordinate eines festen Ursprungsorts (6c) des Signals (6) in der Darstellung (6b) in Abhängigkeit der φ-Koordinate dieses Ursprungsorts (6c) beschreibt, ermittelt wird (121).

7.  Verfahren (100) nach Anspruch 6, **dadurch gekennzeichnet, dass** für die Bahnkurve (6d) ein Ansatz (6e) mit einem Satz freier Parameter (6f) aufgestellt und die Ermittlung (121) des Verlaufs der Amplitude A des Signals (6) für eine Vielzahl von Wertevektoren (6g) der Parameter (6f) wiederholt wird (123).

8.  Verfahren (100) nach Anspruch 7, **dadurch gekennzeichnet, dass** für jeden Wertevektor (6g) der Parameter (6f) die Summe Gsum der Amplituden A des Signals (6) entlang der zugehörigen Bahnkurve (6d) in der Darstellung (6b) des Signals (6) ermittelt wird (122).

9.  Verfahren (100) nach Anspruch 8, **dadurch gekennzeichnet, dass** derjenige Wertevektor (6g), für die die Summe Gsum ihr Maximum Gmax annimmt, als Marker (7) oder als Kandidat (7a) für einen Marker (7) gewertet wird (124), wenn das Verhältnis des Maximums Gmax zum Mittelwert oder Median Gmean aller Amplituden A in der Darstellung (6b) des Signals (6) einen vorgegebenen Schwellwert S erreicht oder überschreitet.

10. Verfahren (100) nach Anspruch 9, **dadurch gekennzeichnet, dass** mindestens zwei über der Laufzeit T bzw. der abgeleiteten Größe q einerseits und über dem Winkel φ andererseits aufgetragene Darstellungen (6b, 6b') von Signalen (6, 6'), die sich auf benachbarte und/oder überlappende Teilvolumina (1b, 1b') des Werkstücks (1) beziehen, ausgewertet werden (125).

11. Verfahren (100) nach Anspruch 10, **dadurch gekennzeichnet, dass** auf einen Marker (7) geschlossen wird (126), wenn die aus den Darstellungen (6b, 6b') ermittelten Kandidaten (7a, 7a') innerhalb eines vorgegebenen Abstands D zueinander liegen.

12. Vorrichtung zur Detektion eines Markers (7), der auf eine Inhomogenität (2a) im Material (2) eines Werkstücks (1) hindeutet, in einem Signal (6), umfassend einen Sender (3) für eine Abfragestrahlung (5) und einen Empfänger (4) für das Signal (6), mit dem das Werkstück (1) auf die Abfragestrahlung (5) antwortet und/oder geantwortet hat, **dadurch gekennzeichnet, dass** Mittel (120) vorgesehen sind, die aus dem Signal (6) zunächst einen in einem Winkel φ periodischen Anteil (6a) abseparieren, der sich bei einer Drehung und/oder Verschwenkung (110) des Werkstücks (1), und/oder des Senders (3), und/oder des Empfängers (4), um den Winkel φ mit dem Winkel φ periodisch ändert, und anschließend aus diesem Anteil (6a) den Marker (7) auswerten.

**Claims**

1.  Method (100) for detecting a marker (7), which indicates an inhomogeneity (2a) in the material (2) of a workpiece (1), in a signal (6) with which the workpiece (1) responds and/or has responded to a receiver (4) in response to interrogation radiation (5) from a transmitter (3),

    **characterized in that** a portion (6a) which is periodic at an angle φ and changes periodically, when the workpiece (1) and/or the transmitter (3)
    and/or the receiver (4) is/are rotated and/or pivoted (110) by the angle φ, with the angle φ, is first separated from the signal (6) and then the marker (7) from this portion (6a) is evaluated (120).

2.  Method (100) according to Claim 1, **characterized in that** a cylindrical workpiece (1) is selected and the rotation (110) takes place or has taken place around the cylinder axis (1a) of the workpiece (1).

3.  Method (100) according to one of Claims 1 to 2, **characterized in that** interrogation radiation (5) is selected, which is reflected at the inhomogeneity (2a) and which is attenuated by the material (2) of the workpiece (1) only to such an extent that the reflected signal (6) can still be detected by the receiver (4).

4.  Method (100) according to one of Claims 1 to 3, **characterized in that** the portion (6a) of the signal (6), which varies with the angle φ, is evaluated (120) from a representation (6b) of the signal (6), in which an amplitude A of the signal (6) is plotted as a function of the angle $\phi$, on the one hand, and a propagation time T of the signal (6), and/or a variable q derived from the propagation time T of the signal (6), on the other hand.

5. Method (100) according to Claim 4, **characterized in that** the distance X between the origin location (6c) of the signal (6) in the workpiece (1) and a predefined location P, at which the signal (6) exits or has exited the workpiece (1), is selected as the derived variable q.

6. Method (100) according to one of Claims 4 to 5, **characterized in that** the course of the amplitude A of the signal (6) along at least one trajectory (6d), which describes the T or q coordinate of a fixed origin location (6c) of the signal (6) in the representation (6b) on the basis of the $\phi$ coordinate of this origin location (6c), is determined (121).

7. Method (100) according to Claim 6, **characterized in that** an approach (6e) with a set of free parameters (6f) is set up for the trajectory (6d) and the determination (121) of the course of the amplitude A of the signal (6) is repeated (123) for a multiplicity of value vectors (6g) of the parameters (6f).

8. Method (100) according to Claim 7, **characterized in that** the sum Gsum of the amplitudes A of the signal (6) along the associated trajectory (6d) in the representation (6b) of the signal (6) is determined (122) for each value vector (6g) of the parameters (6f).

9. Method (100) according to Claim 8, **characterized in that** that value vector (6g) for which the sum Gsum assumes its maximum Gmax is classified (124) as a marker (7) or as a candidate (7a) for a marker (7) if the ratio of the maximum Gmax to the mean value or median Gmean of all amplitudes A in the representation (6b) of the signal (6) reaches or exceeds a predefined threshold value S.

10. Method (100) according to Claim 9, **characterized in that** at least two representations (6b, 6b') of signals (6, 6') that are plotted against the propagation time T or the derived variable q, on the one hand, and against the angle $\phi$, on the other hand, and refer to adjacent and/or overlapping partial volumes (1b, 1b') of the workpiece (1) are evaluated (125).

11. Method (100) according to Claim 10, **characterized in that** a marker (7) is inferred (126) if the candidates (7a, 7a') determined from the representations (6b, 6b') are within a predefined distance D to each other.

12. Device for detecting a marker (7), which indicates an inhomogeneity (2a) in the material (2) of a workpiece (1), in a signal (6), comprising a transmitter (3) for interrogation radiation (5) and a receiver (4) for the signal (6), with which the workpiece (1) responds and/or has responded to the interrogation radiation (5), **characterized in that** means (120) are provided and first separate a portion (6a), which is periodic at an angle $\phi$ and changes periodically, when the workpiece (1) and/or the transmitter (3) and/or the receiver (4) is/are rotated and/or pivoted (110) by the angle $\phi$, with the angle $\phi$, from the signal (6) and then evaluate the marker (7) from this portion (6a).

**Revendications**

1. Procédé (100) de détection d'un marqueur (7), lequel indique une inhomogénéité (2a) dans le matériau (2) d'une pièce à usiner (1), dans un signal (6) au moyen duquel la pièce à usiner (1) répond et/ou a répondu à un récepteur (4) à un rayonnement d'interrogation (5) provenant d'un émetteur (3),

   **caractérisé en ce qu'**à partir du signal (6), une fraction (6a) périodique selon un angle $\phi$ est d'abord séparée, laquelle, lors d'une rotation et/ou d'un pivotement (110) de la pièce à usiner (1) et/ou de l'émetteur (3) et/ou du récepteur (4), varie périodiquement de l'angle $\phi$, avec l'angle $\phi$, et **en ce que** le marqueur (7) est ensuite évalué (120) à partir de cette fraction (6a).

2. Procédé (100) selon la revendication 1, **caractérisé en ce qu'**une pièce à usiner (1) cylindrique est sélectionnée et **en ce que** la rotation (110) s'effectue ou s'est effectuée autour de l'axe du cylindre (1a) de la pièce à usiner (1).

3. Procédé (100) selon l'une quelconque des revendications 1 à 2, **caractérisé en ce qu'**un rayonnement d'interrogation (5) est sélectionné, lequel est réfléchi sur l'inhomogénéité (2a) et n'est atténué par le matériau (2) de la pièce à usiner (1) que dans la mesure où le signal réfléchi (6) peut encore être détecté par le récepteur (4).

4. Procédé (100) selon l'une quelconque des revendications 1 à 3, **caractérisé en ce que** la fraction (6a) du signal (6) variable avec l'angle φ est évaluée (120) à partir d'une représentation (6b) du signal (6) dans laquelle une amplitude A du signal (6) est tracée d'une part, en fonction de l'angle φ, et d'autre part, en fonction d'un temps de propagation T du signal (6) et/ou d'une grandeur q dérivée du temps de propagation T du signal (6).

5. Procédé (100) selon la revendication 4, **caractérisé en ce que** la distance X entre le lieu d'origine (6c) du signal (6) dans la pièce à usiner (1) et un lieu P prédéfini, où le signal (6) sort ou est sorti de la pièce à usiner (1), est sélectionnée comme grandeur dérivée q.

6. Procédé (100) selon l'une quelconque des revendications 4 à 5, **caractérisé en ce que** l'évolution de l'amplitude A du signal (6) est déterminée (121) le long d'au moins une trajectoire (6d), laquelle décrit la coordonnée T ou q d'un lieu d'origine fixe (6c) du signal (6) dans la représentation (6b) en fonction de la coordonnée φ de ce lieu d'origine (6c).

7. Procédé (100) selon la revendication 6, **caractérisé en ce que**, pour la trajectoire (6d), une approche (6e) utilisant un ensemble de paramètres libres (6f) est établie et **en ce que** la détermination (121) de l'évolution de l'amplitude A du signal (6) est répétée (123) pour une pluralité de vecteurs de valeurs (6g) des paramètres (6f).

8. Procédé (100) selon la revendication 7, **caractérisé en ce que**, pour chaque vecteur de valeurs (6g) des paramètres (6f), la somme Gsum des amplitudes A du signal (6) le long de la trajectoire (6d) associée dans la représentation (6b) du signal (6) est déterminée (122).

9. Procédé (100) selon la revendication 8, **caractérisé en ce que** le vecteur de valeurs (6g) pour lequel la somme Gsum atteint son maximum Gmax est considéré comme (124) un marqueur (7) ou comme un candidat (7a) pour un marqueur (7) lorsque le rapport du maximum Gmax à la moyenne ou à la médiane Gmean de toutes les amplitudes A dans la représentation (6b) du signal (6) atteint ou dépasse une valeur seuil S prédéfinie.

10. Procédé (100) selon la revendication 9, **caractérisé en ce qu'**au moins deux représentations (6b, 6b') de signaux (6, 6'), tracées d'une part, en fonction du temps de propagation T ou de la grandeur dérivée q, et d'autre part, en fonction de l'angle φ, et qui concernent des volumes partiels (1b, 1b') adjacents et/ou se chevauchant de la pièce à usiner (1), sont évaluées (125).

11. Procédé (100) selon la revendication 10, **caractérisé en ce qu'**un marqueur (7) est établi (126) lorsque les candidats (7a, 7a') déterminés à partir des représentations (6b, 6b') se situent à une distance D prédéfinie l'un de l'autre.

12. Dispositif de détection d'un marqueur (7), lequel indique une inhomogénéité (2a) dans le matériau (2) d'une pièce à usiner (1), dans un signal (6), comprenant un émetteur (3) destiné à un rayonnement d'interrogation (5) et un récepteur (4) destiné au signal (6) au moyen duquel la pièce à usiner (1) répond et/ou a répondu au rayonnement d'interrogation (5), **caractérisé en ce qu'**il est prévu des moyens (120) qui séparent d'abord du signal (6) une fraction (6a) périodique selon un angle φ, laquelle, lors d'une rotation et/ou d'un pivotement (110) de la pièce à usiner (1) et/ou de l'émetteur (3) et/ou du récepteur (4), varie périodiquement de l'angle φ, avec l'angle φ, et qui évaluent ensuite le marqueur (7) à partir de cette fraction (6a).

# Fig. 1
(Stand der Technik)

# Fig. 2a

# Fig. 2b

**Fig. 3**

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4036005 A1
- EP 1475633 A1
- US 6318178 B1
- WO 2006120875 A1
- DE 19625763 A1
- DE 102009043001 A1
- US 3766387 A
- GB 2521767 A